# EUROPÄISCHE PATENTSCHRIFT

(11) **EP 1 959 912 B1**
(45) Veröffentlichungstag und Bekanntmachung des Hinweises auf die Patenterteilung: **06.01.2010**
(21) Anmeldenummer: 06722517.7
(22) Anmeldetag: 23.02.2006
(51) Int. Cl.: A61K 6/04, C22C 19/07

(54) **DENTALLEGIERUNG UND VERWENDUNG DAFÜR**
DENTAL ALLOY AND USE THEREFOR
ALLIAGE DENTAIRE ET SON UTILISATION

(43) Veröffentlichungstag der Anmeldung: 27.08.2008
(73) Patentinhaber: Petroll, Claudia, 21732 Hollern-Twielenfleth (DE)
(72) Erfinder: Petroll, Claudia, 21732 Hollern-Twielenfleth (DE)
(74) Vertreter: Hansen, Jochen
(86) Internationale Anmeldenummer: PCT/DE2006/000330
(87) Internationale Veröffentlichungsnummer: WO 2007/095872

(56) Entgegenhaltungen:
- EP-A1- 1 666 619
- EP-A1- 1 696 044
- WO-A-00/64403
- WO-A-02/36080
- DE-A1- 3 244 803
- DE-C1- 3 319 457
- DE-C1- 3 415 249
- US-A- 4 483 821
- US-A1- 2005 158 693

## Beschreibung

Die Erfindung betrifft eine Dentallegierung für die Zahnheilkunde sowie die Verwendung dieser Dentallegierung zur spanabhebenden Herstellung von Dentalsuprastrukturen, insbesondere Kronen, Brücken und kombinierter Zahnersatz auf präparierten Pfeilerzähnen und Implantaten.

Dentalsuprastrukturen, also Zahnersatzelemente, sind im Gegensatz zu Dentalimplantaten äußerlich auf entsprechend präparierten Zähnen oder entsprechenden Aufnahmeelementen (abudments) von Dentalimplantaten aufsetzbare Elemente der Zahntechnik. Diese Dentalsuprastrukturen werden im Stand der Technik durch Abdruckabnahme vom Patienten und Abformen über ein Wachsmodell im Wachsausschmelzverfahren hergestellt. Das Wachsausschmelzverfahren ist insbesondere aufgrund des hohen Handarbeitsanteils sehr aufwendig. Darüber hinaus ist das mehrfache Abformen fehlerbehaftet, so dass Maßtoleranzen von 100 µm selten unterschritten werden.

Aus der EP 1 173 136 B1 bzw. der WO 00/64403 der Anmelderin ist eine für das Wachsausschmelzverfahren geeignete Dentalaufbrennlegierung bekannt, die neben Kobalt als Rest bis zu 0,4 % Kohlenstoff, 0,1 bis 1,0 % Mangan, 0,1 bis 1,0 % Silizium, 20 bis 30 % Chrom, bis zu 1 % Nickel, 3 bis 7 % Molybdän, bis zu 0,75 % Eisen, 0,1 bis 5 % Gold und 0,1 bis 4 % Platin enthält. In weiterer Ausgestaltung enthält diese Dentalaufbrennlegierung auch Gallium und/oder Indium in einem Anteil von 0,1 bis 16 %. Diese Legierung ist speziell auf das Wachsausschmelzverfahren abgestimmt.

Aus der DE 34 15 249 C1 ist darüber hinaus eine Dentalaufbrennlegierung auf Basis von Chrom und Kobalt bekannt, die 1 bis 3 % Mangan, 1 bis 2,5 % Silizium, 6 bis 10 % Wolfram, 0,5 bis 1,5 % Niob, 0,5 bis 1 ,5 % Tantal, 1 bis 3 % Iridium sowie Zirkonium und/oder Cer und/oder Yttrium in der Summe von 0,2 bis 0,08 % enthält. In weiterer Ausgestaltung kann diese Legierung bis zu 1 % Aluminium enthalten. Diese Legierung ist geeignet zur Herstellung von Teilen für den Zahnersatz durch Gussverfahren.

Eine weitere Dentalaufbrennlegierung ist aus der DE 33 19 457 C1 bekannt, bestehend aus 42 bis 69,5 Gew.-% Kobalt, 10 bis 35 Gew.-% Chrom und 5 bis 25 Gew.-% Wolfram, wobei sie zusätzlich 2 bis 10 Gew.-% Eisen, 1 bis 4 Gew.-% Aluminium, 0 bis 5 Gew.-% Rhenium und 0 bis 2 Gew.-% Titan enthalten kann, wobei die Summe der Gehalte von Chrom, Wolfram und Rhenium mindestens 27,5 Gew.-% und höchstens 45 Gew.-% beträgt und die Summe an Titan und Aluminium höchstens 5 Gew.-% ergibt.

Ferner ist von der KaVo Dental GmbH, 88400 Biberach ein keramisches Material für Dentalsuprastrukturen, insbesondere Vollkronen unter der Bezeichnung "KaVo Everest BIO HPC-Blank" bekannt, bei dem es sich im wesentlichen um eine Zirkonsilikatkeramik handelt. Dieses Material wird im weichen Grünzustand spanabhebend in die gewünschte Form gebracht und anschließend bei ca. 1500 °C gesintert. Bei derartigen Keramikkronen, auch aus Zirkonoxid und Aluminiumoxid (Al₂O₃) besteht der Nachteil, dass dieses Material eine sehr niedrige Bruchdehnung nahe 0 hat. Aufgrund dieser hohen Steifigkeit werden damit die bei Kaubewegungen auftretenden hohen Lastspitzen über die künstliche Struktur im wesentlichen ungedämpft auf den Kiefer des Patienten übertragen. In der Folge werden die Sharpischen Fasern, die den Zahn in klinische Krone und anatomische Krone trennen und für einen dauerhaften Halt des Zahnes im Kiefer mitverantwortlich sind, stark und ggf. übermäßig beansprucht.

Ferner ist nachteilig, dass bei der spanabhebenden Bearbeitung dieser Keramik Wandstärken von mindestens 0,6 mm stehen gelassen werden müssen. Entsprechend ist der Zahn für eine aus dieser Keramik hergestellten Krone wenigstens um diese Wandstärkengröße zuzüglich Toleranz und Zementschicht abzutragen, womit ggf. eine Beeinflussung der Pulpa und eine damit zusammenhängende Empfindlichkeit für den Patienten entstehen könnte. Entsprechend ist die Indikation bei kombinierten prothetischen Versorgungen, insbesondere bei festsitzendem Zahnersatz, stark eingeschränkt.

Ferner ist nachteilig, dass die Keramik lediglich in einem nicht zu Ende geführten "halbgesinterten" Zustand spanabhebend bearbeitet werden kann. Zur endgültigen Ausbildung des Gefüges ist somit ein nachgeschaltetes Sinterverfahren erforderlich. Bei der thermischen Beanspruchung durch das abschließende Sintern bei beispielsweise 1500 °C können Schrumpfungen entstehen, die die Passgenauigkeit des Zahnersatzes beeinträchtigen. Somit ist die technische Einsetzbarkeit dieser Keramik begrenzt.

Gleichwohl weist eine spanabhebende Herstellung grundsätzlich eine hohe Maßgenauigkeit auf. Ferner ist das keramische Material sehr kostengünstig und die Fertigung einfacher als beim Wachsausschmelzverfahren. Deshalb besteht in der Zahntechnik ein Bedarf an automatisiert herstellbaren Zahnersatzelementen. Bisher waren jedoch metallische Materialien der Zahntechnik für eine spanabhebende Herstellung nicht geeignet.

Aufgrund der erheblichen Fertigungsvorteile bei einer spanabhebenden Herstellung von Dentalsuprastrukturen sowohl aus Kostenaspekten wie auch aus Maßhaltigkeitsgründen, ist es Aufgabe der Erfindung ein für die spanabhebende Herstellung und für den Zahnersatz geeignetes Material anzugeben, das die vorgenannten Nachteile nicht aufweist bzw. verringert.

Erfindungsgemäß wird diese Aufgabe gelöst durch eine Dentallegierung auf Basis einer Edelmetall freien Legierung.

Für die vorliegende Erfindung beziehen sich die Prozentangaben auf den Gewichtsanteil der jeweiligen Komponente bezogen auf die Gesamtlegierung.

Die Stoffzusammensetzung basiert auf einer Universal-Legierung ähnlich der aus der EP 1 173 136 B1 bekannten Dentalaufbrennlegierung. Überraschenderweise konnten durch Zusatz von Wolfram und Aluminium sehr gute Zerspanungseigenschaften erreicht werden, wobei die übrigen positiven Eigenschaften der bekannten Legierung im wesentlichen erhalten bleiben. Im Gegensatz zur bekannten Dentalaufbrenniegierung konnte auf die Elemente Platin und Gold vollständig verzichtet werden, womit die Materialkosten verringert sind.

Die erfindungsgemäße Dentallegierung zeichnet sich neben den sehr guten Zerspanungseigenschaften auch dadurch aus, dass das Material bei der CNC-Bearbeitung bis auf eine Wandstärke von 0,2 mm ausgearbeitet werden kann. Die Beeinträchtigung eines mit einer Krone zu versehenden Zahnes aufgrund des erforderlichen Materialabtrags wird somit gegenüber dem bekannten spanabhebend zu bearbeiteten Keramikmaterial halbiert. Dabei ist herauszustellen, dass grazilere zervikale Zahnfleischsäume von Kronen der Kosmetik und Ästhetik dienen. Sie wirken aber insbesondere der Resorption des Zahnfleisches entgegen und verhindern Schmutzansammlungen im Sulcus. Die resultierenden Randspalten zum klinischen Zahn an der Präparationsgrenze sind in Verbindung mit Zement oder Klebwerkstoffen bakteriendicht. Sie werden beim Tragen nicht ausgewaschen (Sekundärkaries).

Mit der erfindungsgemäßen Legierung lässt sich somit jede sinnvolle Konstruktionsidee, die prothetisch abgefragt wird, ausführen. Die Härte, Spanbarkeit und die Zähelastizität sind in harmonischen Einklang. Filigranste Konstruktionen lassen sich aus den Blanks der Legierung mit Genauigkeiten ≤ 20 µm spanabhebend, insbesondere mit 5-achsigen CNC-Maschinen werkzeugschonend herstellen.

Bei der Verarbeitung lassen sich durch Verwendung von hochreinen Ausgangsstoffen und durch Einstellen eines extrem feinkristallinen Gefüges hoch zähelastische Legierungen erhalten, die ohne Probleme spanabhebend bearbeitet werden können und zudem außerordentlich gut polierbar sind. Polierte Oberflächen verhindern Plaqueansammlungen.

Ferner ist es vorteilhaft, dass bei der spanabhebenden Bearbeitung von aus dieser Legierung erzeugten Gusshalbzeugen (Blanks) extrem kurze (runde) Späne erzeugt werden. Dieses Verhalten ist Basis für die Bearbeitbarkeit der Legierung mit NC/CNC-Fräsmaschinen. Das aus dem Blank gefertigte prothetische Teil hat somit seine endgültige Konfiguration.

Die erfindungsgemäße EMF-Legierung (edelmetallfreie Legierung) bildet quasi eine Knetlegierung mit für den Einsatzzweck hervorragend angepassten Eigenschaften. In der Schmelze bildet sich eine puddingartige Konsistenz, die sich in den in Gussformen erstarrten Blanks durch abgerundete Kanten und Ecken abbildet. Besonders hervorzuheben ist, dass beim Erstarren der Schmelze nur eine äußerst niedrige Volumenkontraktion von ca. 1,8 Vol% festzustellen ist. Diese äußerst niedrige Volumenkontraktion dürfte auch dafür verantwortlich sein, dass das erstarrte Material in den Blanks lunkerfrei auftritt. Mithin ist das für die spanabhebende Bearbeitung vorbereitete Halbzeug in Form des Gussblocks praktisch fehlerfrei, so wie dies in Schnitten und durch Röntgenuntersuchungen belegt werden konnte.

Ferner hat sich bei Versuchen gezeigt, dass die erfindungsgemäße Legierung eine für die Patientengesundheit wichtige sehr niedrige Oxidationsrate zeigt. Im sog. "7-day-test" unterschreitet die Oxidationsrate den gemeinschaftsweit geltenden Grenzwert von 100 µg pro 7 Tage deutlich.

Ferner wird darauf hingewiesen, dass die erfindungsgemäße Dentallegierung neben den in den Ansprüchen genannten Bestandteilen auch Spuren von Verunreinigungen enthalten kann, wie sie z. B. in den Ausgangsmaterialien vorhanden sein können oder im Zuge der Herstellung eingeschleppt werden können.

Das offenbar für die sehr gute Zerspanbarkeit besonders wichtige Element Wolfram sollte in einer bevorzugten Ausgestaltung der Dentallegierung in einem Anteil von 2,5 bis 10 % vorliegen.

Vorzugsweise ist Molybdän in einem Gewichtsanteil von 3 bis 8 % und/oder Mangan in einem Anteil von 0,1 bis 1 % und/oder Silizium in einem Anteil von 0,2 bis 4 % in der Legierung enthalten.

Durch die ergänzende Zugabe von Metallen der seltenen Erden, nämlich Cer und/oder Yttrium, sowie den weiteren Elementen Tantal, Niob und/oder Zirkon wird eine Erhöhung der Haftfestigkeit der vorliegenden Dentallegierung im Metall-Keramik-Verbund erreicht. Ebenso dient der Anteil von 0,5 bis 2 % Zinn der Verbesserung der Haftung zwischen der vorliegenden Dentallegierung und daran anzuhaftendem Keramikmaterial.

Die erfindungsgemäßen Legierungen können ohne weiteres mit herkömmlichen handelsüblichen Bondermaterialien (Haftgrundvermittlern) verarbeitet werden. Ein besonders geeigneter Haftgrundvermittler ist aus der DE 100 22 559 B4 der Anmelderin bekannt.

Die Legierungen eigenen sich zur Herstellung von beliebigen Dentalsuprastrukturen, wie beispielsweise Kronen, Brücken, kombiniertem Zahnersatz etc. Sie können auch hervorragend für die Herstellung von Dentalsuprastrukturen mit entsprechenden Beschichtungen wie z. B. Feldspat-Keramiken und/oder Hydrothermalen Nano Leuzit-Keramiken verwendet werden.

Ein besonderer Vorteil der erfindungsgemäßen Legierung ist ihre leichte Handhabbarkeit bei der Herstellung der Gußhalbzeuge und die sehr gute spanabhebende Verarbeitbarkeit. Beim Herstellen der Schmelze in Blank-Form bildet sie keine Schlacken aus und aufgrund der Oxidreduzierung durch Impfen der Form (Keimbildner) wird ein Ankleben der Schmelze an die Gussform verhindert.

Die physikalischen und chemischen Eigenschaften konnten gegenüber bekannten EMF-Dentalaufbrennlegierungen gehalten werden. Die mechanischen Eigenschaften erlauben erstmals die Zerspanung mit NC/CNC-Maschinen. Gegenüber keramischem Material zeichnet sich die erfindungsgemäße Dentallegierung in Abhängigkeit von der Gussführung mit einer Bruchdehnung bis zu 20 % aus.

Für die erfindungsgemäßen Legierungen sind zudem keine allergenen Reaktionen bekannt.

Ein generelles Problem bei der Herstellung von Metall-Keramik-Verbünden ist das in der Regel unterschiedliche Wärmeausdehnungsverhalten der Metalllegierung einerseits und des Keramikmaterials andererseits, aufgrund dessen Sprünge oder Abplatzungen auftreten können. Gewünscht ist jedoch ein Verbund zwischen Metall und Keramik von möglichst unbegrenzter Dauer, der nicht durch Sprünge oder Abplatzungen in der Keramik zerstört wird.

Es hat sich gezeigt, dass sich der Wärmeausdehnungskoeffizient der erfindungsgemäßen Legierungen ohne weiteres auf einen Wert einstellen lässt, der dem Wärmeausdehnungskoeffizienten der üblicherweise verwendeten hochschmelzenden Keramiken entspricht, der in der Größenordnung um 13,9 x 10⁻⁶ (bei 500 °C; gemäß DIN EN ISO 9693) liegt.

Gemäß einer besonders bevorzugten Ausführungsform weist daher die erfindungsgemäße Legierung einen Wärmeausdehnungskoeffizienten auf, der dem Wärmeausdehnungskoeffizienten des verwendeten Keramikmaterials gleich oder ähnlich ist. Vorzugsweise hat die erfindungsgemäße Legierung einen Wärmeausdehnungskoeffizienten von 13,9 x 10⁻⁶ bis 15,0 x 10⁻⁶, insbesondere in etwa 14,7 x 10⁻⁶ (bei 500 °C).

Insgesamt wird mit der erfindungsgemäßen Zusammensetzung der Dentallegierung ein Ausschluss von Mikrolunkern und Sauglunkern in den aus der Schmelze hergestellten Halbzeugen (Blanks) erreicht, wie röntgenologische Untersuchungen zeigen. Die flüssige, puddingartige Schmelze trägt dazu bei, einen exzellenten Formfüllungsgrad der Schmelze in der Form herbeizuführen. Bevorzugt wird die Schmelze im sogenannten roll-over-Verfahren in die Form gebracht. Hieraus resultiert eine feindentritische Kristallstruktur. Die schnelle Erstarrung der Schmelze verhindert darüber hinaus die Lunkerbildung. Bereits bei der Urschmelze gab es keinen Ausschuss. Damit ist eine hohe Wirtschaftlichkeit der Schmelzausbringung zu erwarten.

## Patentansprüche

1. Dentallegierung bestehend aus
20 bis 35 % Chrom,
0 bis 10 % Molybdän,
0 bis 3 % Mangan,
0,5 bis 2 % Zinn,
2 bis 10 % Gallium,
2 bis 8 % Indium,
0 bis 8 % Silizium,
1,5 bis 15 % Wolfram,
0,1 bis 2 % Aluminium,
Kobalt Rest sowie Verunreinigungen.

2. Dentallegierung nach Anspruch 1, **gekennzeichnet durch** einen Anteil von 3 bis 8 % Molybdän.

3. Dentallegierung nach Anspruch 1 oder 2, **gekennzeichnet durch** einen Anteil von 0,1 bis 1 % Mangan.

4. Dentallegierung nach Anspruch 1, 2 oder 3, **gekennzeichnet durch** einen Anteil von 0,2 bis 4 % Silizium.

5. Dentallegierung nach einem der vorangehenden Ansprüche, **gekennzeichnet durch** einen Anteil von 2,5 bis 10 % Wolfram.

6. Dentallegierung nach einem der vorangehenden Ansprüche, **gekennzeichnet durch** einen Anteil von 5 bis 8 % Gallium.

7. Dentallegierung nach einem der vorangehenden Ansprüche, zusätzlich mindestens ein Element enthaltend, ausgewählt aus Tantal, Niob, Cer, Yttrium und Zirkon mit einem Gesamtanteil von 0,1 bis 3 %.

8. Dentallegierung nach einem der vorangehenden Ansprüche, wobei die Legierung einen Wärmeausdehnungskoeffizienten von 13,9 x 10⁻⁶ bis 15,0 x 10⁻⁶ (bei 500 °C) hat.

9. Verwendung einer Dentallegierung nach einem der vorangehenden Ansprüche zur spanabhebenden Herstellung von Dentalsuprastrukturen, insbesondere Kronen, Brücken und kombinierter Zahnersatz.

## Claims

1. A dental alloy consisting of
20 to 35% chromium,
0 to 10% molybdenum,
0 to 3% manganese,
0.5 to 2% tin,
2 to 10% gallium,
2 to 8% indium,
0 to 8% silicon,
1.5 to 15% tungsten,
0.1 to 2% aluminium,
cobalt as the remainder as well as impurities.

2. A dental alloy according to claim 1, **characterised by** a content of 3 to 8% molybdenum.

3. A dental alloy according to claim 1 or 2, **characterised by** a content of 0.1 to 1% manganese.

4. A dental alloy according to claim 1, 2 or 3, **characterised by** a content of 0.2 to 4% silicon.

5. A dental alloy according to any one of the preceding claims, **characterised by** a content of 2.5 to 10% tungsten.

6. A dental alloy according to any one of the preceding claims, **characterised by** a content of 5 to 8% gallium.

7. A dental alloy according to any one of the preceding claims, additionally containing at least one element selected from tantalum, niobium, cerium, yttrium and zirconium with a total content of 0.1 to 3%.

8. A dental alloy according to any one of the preceding claims, wherein the alloy has a coefficient of thermal expansion of 13.9 x 10⁻⁶ to 15.0 x 10⁻⁶ (at 500°C).

9. Use of a dental alloy according to any one of the preceding claims for the manufacture, through cutting, of dental superstructures, in particular crowns, bridges and a combined dental prosthesis.

## Revendications

1. Alliage dentaire composé de
20 à 35 % de chrome,
0 à 10 % de molybdène,
0 à 3 % de manganèse,
0,5 à 2 % d'étain,
2 à 10 % de gallium,
2 à 8 % d'indium,
0 à 8 % de silicium,
1,5 à 15 % de tungstène,
0,1 à 2 % d'aluminium,
le reste étant du cobalt ainsi que des impuretés.

2. Alliage dentaire selon la revendication 1, **caractérisé par** une teneur en molybdène comprise entre 3 et 8 %.

3. Alliage dentaire selon la revendication 1 ou 2, **caractérisé par** une teneur en manganèse comprise entre 0,1 et 1 %.

4. Alliage dentaire selon la revendication 1, 2 ou 3, **caractérisé par** une teneur en silicium comprise entre 0,2 et 4 %.

5. Alliage dentaire selon l'une des revendications précédentes, **caractérisé par** une teneur en tungstène comprise entre 2,5 et 10 %.

6. Alliage dentaire selon l'une des revendications précédentes, **caractérisé par** une teneur en gallium comprise entre 5 et 8 %.

7. Alliage dentaire selon l'une des revendications précédentes comprenant au moins un élément supplémentaire choisi parmi le tantale, le niobium, du cerium, de l'yttrium et du zirconium avec une teneur comprise entre 0,1 et 3 %.

8. Alliage dentaire selon l'une des revendications précédentes, lequel a un coefficient de dilatation thermique compris entre 13,9 x 10⁻⁶ et 15,0 x 10⁻⁶ (à 500 °C).

9. Utilisation d'un l'alliage dentaire selon l'une des revendications précédentes, pour la fabrication par enlèvement de copeaux de suprastructures dentaires, notamment de couronnes, de bridges et de prothèses dentaires combinées.
